# EUROPEAN PATENT APPLICATION

(11) **EP 2 072 492 A1**
(43) Date of publication of application: **24.06.2009**
(21) Application number: 07254896.9
(22) Date of filing: 17.12.2007
(51) Int. Cl.: C07C 31/08, C07C 69/14, C07C 67/37, C07C 43/04, C07C 41/09, C07C 29/149, C07C 29/151

(54) **Process for the conversion of hydrocarbons to ethanol**

(71) Applicant: BP p.l.c., London SW1Y 4PD (GB)
(72) Inventor: The designation of the inventor has not yet been filed
(74) Representative: De Kezel, Eric

(57) **Abstract**

The present invention relates to a process for the conversion of a carbonaceous feedstock to ethanol. via the formation of syngas, methanol, dimethyl ether and methyl acetate.

## Description

The present invention relates to a process for the conversion of a carbonaceous feedstock to alcohol.

In recent years increased use and demand for alcohols such as methanol, ethanol and higher alcohols has led to a greater interest in processes relating to alcohol production. The said alcohols may be produced by the fermentation of, for example, sugars and/or cellulosic materials.

Alternatively alcohols, such as ethanol, may be produced from synthesis gas. Synthesis gas refers to a combination of hydrogen and carbon oxides produced in a synthesis gas plant from a carbon source such as natural gas, petroleum liquids, biomass and carbonaceous materials including coal, recycled plastics, municipal wastes, or any organic material. Thus, alcohol and alcohol derivatives may provide non-petroleum based routes for the production of valuable chemicals and fuels.

Generally, the production of alcohols, for example methanol, takes place via three process steps: synthesis gas preparation, methanol synthesis, and methanol purification. In the synthesis gas preparation step, an additional stage maybe employed whereby the feedstock is treated, e.g. the feedstock is purified to remove sulphur and other potential catalyst poisons prior to being converted into synthesis gas. This treatment can also be conducted after synthesis gas preparation; for example, when coal or biomass is employed.

The reaction to produce alcohol(s) from synthesis gas is generally exothermic. The formation of C2 and C2+ alcohols is believed to proceed via the formation of methanol for modified methanol catalysts and cobalt molybdenum sulphide catalysts. However, the production of methanol is equilibrium limited and thus requires high pressures in order to achieve viable yields. Hence, pressure can be used to increase the yield, as the reaction which produces methanol exhibits a decrease in volume, as disclosed in U.S. Pat. No.3,326,956. Improved catalysts have now allowed viable rates of methanol formation to be achieved at reduced reaction temperatures, and hence allow commercial operation at lower reaction pressures, e.g. a copper oxide-zinc oxide-alumina catalyst that typically operates at a nominal pressure of 5-10 MPa and temperatures ranging from approximately 150 °C. to 450 °C. over a variety of catalysts, including CuO/ZnO/A12 03, CuO/ZnO/Cr2 03, ZnO/Cr2O3, and supported Fe, Co, Ni, Ru, Os, Pt, and Pd catalysts. A low-pressure, copper-based methanol synthesis catalyst is commercially available from suppliers such as BASF, ICI Ltd. of the United Kingdom, and Haldor-Topsoe. Methanol yields from copper-based catalysts are generally over 99.5% of the converted CO+CO2 present. Water is a by-product of the conversion of CO2 to methanol and the conversion of CO synthesis gas to C2 and C2+ oxygenates. In the presence of an active water gas-shift catalyst, such as a methanol catalyst or a cobalt molybdenum catalyst the water equilibrates with the carbon monoxide to give CO2 and hydrogen. A paper entitled, "Selection of Technology for Large Methanol Plants," by Helge Holm-Larsen, presented at the 1994 World Methanol Conference, Nov. 30-Dec. 1, 1994, in Geneva, Switzerland, reviews the developments in methanol production and shows how further reduction in costs of methanol production will result in the construction of very large plants with capacities approaching 10,000 metric tonnes per day.

Other processes, for the production of C2 and C2+ alcohol(s), include the processes described hereinafter; US 4,122,110 relates to a process for manufacturing alcohols, particularly linear saturated primary alcohols, by reacting carbon monoxide with hydrogen at a pressure between 20 and 250 bars and a temperature between 150 and 400 °C., in the presence of a catalyst, characterized in that the catalyst contains at least 4 essential elements: (a) copper (b) cobalt (c) at least one element M selected from chromium, iron, vanadium and manganese, and (d) at least one alkali metal.

US 4,831,060 relates to the production of mixed alcohols from carbon monoxide and hydrogen gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulfiding. The composition of the mixed alcohols fraction can be selected by selecting the extent of intimate contact among the catalytic components.

Journal of Catalysis 114, 90-99 (1988) discloses a mechanism of ethanol formation from synthesis gas over CuO/ZnO/Al2O3. The formation of ethanol from carbon monoxide and hydrogen over a CuO/ZnO methanol catalyst is studied in a fixed-bed microreactor by measuring the isotopic distribution of the carbon in the product ethanol when isotopically-enriched C¹³ methanol was added to the feed.

As the importance of ethanol is ever increasing in today's world, so is the need and desire to produce ethanol with a higher carbon efficiency, a higher conversion and an improved selectivity from a carbonaceous feedstock. Hence, the present invention provides a process that allows one to produce ethanol from a carbonaceous feedstock, with improved carbon efficiency, a higher selectivity and in particular, with a more efficient conversion to ethanol.

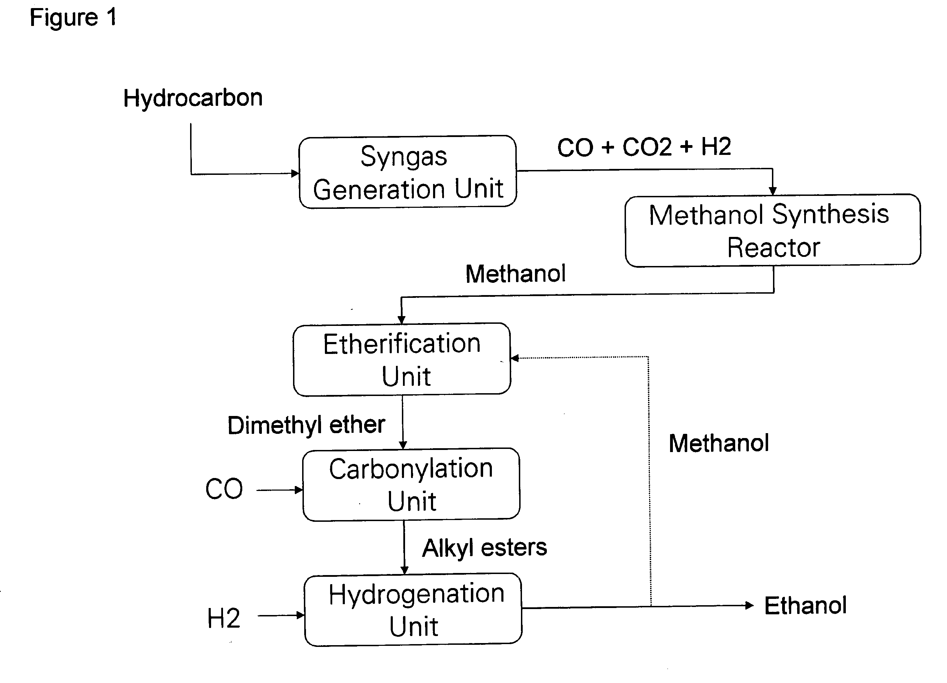

Figure 1 represents an embodiment of a process scheme according to the present invention, wherein the references correspond to those used in the present description and appending claims.

Thus, the present invention relates to a process for the conversion of methanol to ethanol, characterised by the following steps:
1. introducing methanol into an etherification unit to produce dimethyl ether,
2. introducing at least a part of the dimethyl ether from step 1, together with carbon monoxide and optionally hydrogen, into a carbonylation unit, in the presence of an acidic homogeneous or heterogeneous carbonylation catalyst, to produce methyl ethanoate,
3. introducing at least a part of the methyl ethanoate from step 2, together with hydrogen, into a hydrogenation unit, to produce methanol and ethanol,
4. optionally recycling at least part of the methanol from step 3, into the etherification unit of step 1, and
5. recovering ethanol from step 3.

Furthermore, the present invention relates to a process for the conversion of a hydrocarbon feedstock into ethanol, wherein the hydrocarbons are first converted into synthesis gas, which is subsequently converted into ethanol, characterised by the following steps:
1. introducing a hydrocarbon feedstock, into a synthesis gas generation unit, in order to produce a mixture of carbon oxide(s) and hydrogen (synthesis gas),
2. introducing at least a part of the carbon oxide(s) and hydrogen, from step 1, into a methanol synthesis unit, to produce methanol,
3. introducing at least a part of the methanol from step 2, into an etherification unit to produce dimethyl ether,
4. introducing at least a part of the dimethyl ether from step 3, together with carbon monoxide and optionally hydrogen, into a carbonylation unit, in the presence of a heterogeneous carbonylation catalyst, to produce methyl ethanoate,
5. introducing at least a part of the methyl ethanoate from step 4, together with hydrogen, into a hydrogenation unit, to produce methanol and ethanol,
6. optionally recycling at least part of the methanol, from step 5, into the etherification unit of step 3, and
7. recovering the ethanol from step 5.

According to one aspect of the present invention, the synthesis gas feedstock, a mixture of carbon oxide(s) and hydrogen, that is used to produce the methanol feed stream, is preferably produced from a hydrocarbon feedstock.

The hydrocarbon feedstock is preferably a carbonaceous material, for example biomass, plastic, naphtha, refinery bottoms, crude synthesis gas (from underground coal gasification or biomass gasification), smelter off gas, municipal waste, coal bed methane, coal, and/or natural gas, with coal and natural gas being the preferred sources, and natural gas being the most preferable source. To one skilled in the art a combination of sources can also be used, for example coal and natural gas to advantageously increase the hydrogen to carbon ratio.

Natural gas commonly contains a range of hydrocarbons (e.g. C1-C3 alkanes), in which methane predominates. In addition to this, natural gas will usually contain nitrogen, carbon dioxide and sulphur compounds. Preferably the nitrogen content of the feedstock is less than 40 wt %, more preferably less than 10 wt % and most preferably less than 1 wt %.

Processes for producing mixtures of carbon oxide(s) and hydrogen (commonly known as synthesis gas), in a synthesis gas generation unit (e.g. a synthesis gas reactor), are well known. Each method has its advantages and disadvantages, and the choice of using a particular reforming process over another is governed by economic and available feed stream considerations, as well as by the desire to obtain the optimum (H2-CO2):(CO+CO2) molar ratio in the resulting synthesis gas that is suitable for further chemical processing. A discussion of the available synthesis gas production technologies is provided in both "Hydrocarbon Processing" V78, N.4, 87-90, 92-93 (April 1999) and "Petrole et Techniques", N. 415, 86-93 (July-August 1998), and are both hereby incorporated by reference.

It is also known that the synthesis gas may be obtained by catalytic partial oxidation of hydrocarbons in a microstructured reactor as exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438. The synthesis gas can also be obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

Typically, for commercial synthesis gas production the pressure at which the synthesis gas is produced from a steam reformer ranges from approximately 0.1 to 10 MPa, preferably 2 to 3 MPa, and the temperatures at which the synthesis gas exits the reformer ranges from approximately 700 to 1000 °C. Likewise, for commercial synthesis gas production the pressure at which the synthesis gas is produced from an auto-thermal reformer ranges from approximately 0.1 to 10 MPa, preferably 2 to 5 MPa, and the temperatures at which the synthesis gas exits the reformer ranges from approximately 700 to 1300 °C. Where the high temperatures are necessary in order to produce a favourable equilibrium for synthesis gas production, and to avoid metallurgy problems associated with carbon dusting. The synthesis gas contains a molar ratio of (H2-CO2):(CO+CO2) ranging from 0.8 to 3.0, which is dependent on the hydrocarbon feedstock(s) and the method of reforming used. For example, when natural gas is used as the hydrocarbon feedstock for steam reforming, the synthesis gas obtained usually has a maximum (H2-CO2):(CO+CO2) ratio of 3.0. However, when natural gas is used as the hydrocarbon feedstock for auto-thermal reforming, the synthesis gas obtained usually has a (H2-CO2):(CO+CO2) ratio of 1.5.

According to a preferred embodiment of the present invention, when ethanol (and optionally methanol) are the targeted products, the molar ratio, (H2-CO2):(CO+CO2), of the synthesis gas stream exiting the synthesis gas generation unit(s) is greater than 1.6, more preferably greater than 1.8, and most preferably greater than 2.0. Preferably, the molar ratio, (H2-CO2):(CO+CO2), of said synthesis gas stream exiting the synthesis gas generation unit(s) is less than 3.0, preferably less than 2.75, more preferably less than 2.4 and most preferably less than 2.2.

Furthermore, since the present invention allows to target multiple products, such as methyl ethanoate and/or ethanoic acid (via hydrolysis of the alkyl esters) together with said ethanol (and optional methanol) the (H2-CO2):(CO+CO2) of the synthesis gas required to synthesise the said ester and/or acid products is preferably lower than the aforementioned values.

According to another embodiment of this invention when the hydrocarbon feed stock used for synthesis gas generation is not a aliphatic hydrocarbon (e.g. coal, aromatic material, biomass e.g. fermentation residues) the molar ratio (H2-CO2):(CO+CO2) of the exit synthesis gas is preferably adjusted to the target value by addition of hydrogen and/or removal of CO2.

CO2 may be removed by the use of a simple, yet effective, separation method known to those skilled in the art, for example, a "membrane separation method". Such membrane technologies can be found in 'Purification and Recovery Options for Gasification' D. J. Kubek, E. Polla, F. P. Wilcher, UOP, 1996, and are hereby incorporated by reference.

Alternatively, CO2 may be recovered and removed by any suitable method(s) known to those skilled in the art, for example, by reacting with amines; performing a methanol wash (i.e. the RECTISOL™ process) and/or by using hot potassium carbonate (e.g. the BENFIELD™ process).

According to a preferred embodiment of the present invention, the exit stream obtained from the synthesis gas generation unit (e.g. using a steam reformer), comprises essentially a mixture of carbon oxide(s) and hydrogen. It can also comprise water, nitrogen and traces of unconverted hydrocarbons (e.g. C1-C3 alkanes).

According to a preferred embodiment of the present invention, during synthesis gas generation, an additional stage may be employed whereby the feedstock is first purified to remove sulphur and other potential catalyst poisons (such as halides or metals e.g. Hg) prior to being converted into synthesis gas; alternatively this treatment can also be performed after synthesis gas preparation for example, when coal or biomass are used.

According to an embodiment of the present invention, at least part of the said synthesis gas stream is then introduced into a methanol synthesis unit, in order to produce a stream comprising methanol. Preferably the molar ratio, (H2-CO2):(CO+CO2), of said synthesis gas feed stream fed into the methanol synthesis unit is greater than 1.6, more preferably greater than 1.8 and most preferably greater than 2.0. Preferably the molar ratio, (H2-CO2):(CO+CO2), of said synthesis gas feed stream fed into the methanol synthesis unit is less than 3.0, more preferably less than 2.5 and most preferably less than 2.2.

According to a preferred embodiment of the present invention, the methanol synthesis unit may be any unit that is suitable for producing methanol, for example a fixed bed reactor, which can be run with or without external heat exchange equipments e.g. a multi-tubular reactor; or a fluidised bed reactor; or a void reactor.

Preferably the methanol synthesis unit is operated at a temperature of greater than 200°C, more preferably greater than 220°C and most preferably greater than 240°C; and preferably less than 310°C, more preferably less than 300 °C and most preferably less than 290°C. Preferably the methanol synthesis unit is operated at pressure of greater than 2 MPa and most preferably greater than 5 MPa; and preferably less than 10 MPa and most preferably less than 9MPa. In fact, since methanol synthesis is an exothermic reaction, the chosen temperature of operation is governed by a balance of promoting the forward reaction (i.e. by not adversely affecting the equilibrium) and aiding the rate of conversion (i.e. higher productivity).

The catalysts used for methanol synthesis can be divided into 2 groups:
i. the high pressure zinc catalysts, composed of zinc oxide and a promoter; and
ii. low pressure copper catalysts, composed of zinc oxide, copper oxide and a promoter.

Hence, according to a preferred embodiment of the present invention, the preferred methanol synthesis catalyst is a mixture of copper, zinc oxide, and a promoter such as, chromia or alumina. Under the aforementioned operating conditions, these said mixtures can catalyse the production of methanol from carbon monoxide and hydrogen with a high selectivity.

Additionally by-products such as methane, ethanol and other higher alcohols may also be produced during methanol synthesis. According to a preferred embodiment of this aspect of the present invention, the stream exiting the methanol synthesis reactor is subsequently purified to remove said by-products by any methods known to those in the art.

According to the present invention, methanol is introduced into an etherification unit to produce dimethyl ether. Preferably, said methanol introduced into the etherification unit emanates from the aforementioned methanol synthesis unit; however, the methanol may also be produced by any other appropriate method known to those skilled in the art, e.g. via wood pyrolysis.

For example, dimethyl ether is commonly produced by the Lewis or Bronsted acid catalysed etherification of methanol; numerous catalysts for this reaction have been reported e.g. Houben-Weyl, vol. VI/3, part 3, Georg Thieme Verlag, Stuttgart 1965, pp. 17, 18. Dr. Alexander Wacker, Gesellschaft für elektrochemische Industrie GmbH, DE 680 328, 1934 (P. Halbig, O. Moldenhauer). R. L. Brown, W. W. Odells, US 1 873 537, 1927. N. V. de Bataafsche Petroleum Maatschappij, FR 701 335, 1930; GB 332 756, 1929;GB 350 010, 1931;GB 403 402, 1932. Further examples of etherification catalysts include: iron chloride; copper sulphate; copper chloride; manganese chloride; aluminium chloride; aluminium sulphate; chromium sulphate; alums; thorium compounds; aluminium oxide; titanium oxide; barium oxide; silica gel; aluminium phosphate; and acidic ionic liquids. The chosen etherification catalyst may be either homogeneous or heterogeneous.

According to the present invention the preferred etherification catalysts are heterogeneous catalysts, such as aluminium oxides and aluminium silicate, which can be modified by doping. Corresponding etherification catalysts and process operating conditions that may be advantageously used according to the present invention are described in Mobil Oil Corporation, DE 2 818 831, 1978 (F. G. Dwyer, A. B. Schwartz); DE-OS 3 201 155, 1982 (W. K. Bell, C. Chang); Du Pont, EP-A 99 676, 1983 (D. L. Brake); Mitsubishi Chemical Industries, EP-A 124 078, 1984 (N. Murai, K. Nakamichi, M. Otake, T. Ushikubo).

Zeolites, strong acid ion exchange resins, supported heteropolyacids (such as silicotungstenic acid) and mixtures thereof, can also advantageously be used as etherification catalysts; wherein supported heteropolyacids catalysts are preferably used according to the present invention.

During the methanol etherification process to produce dimethyl ether (DME), water is also produced. It is preferred according to the present invention to proceed with the removal of said water prior to introduction of the DME to the next stage of this invention.

According to the present invention, at least a part of the aforementioned DME together with carbon monoxide, and optionally hydrogen, are introduced into a carbonylation unit in the presence of an acidic homogeneous or heterogeneous carbonylation catalyst. The applicants have found a preferred embodiment, whereby it is especially advantageous (e.g. increased catalyst lifetime) to conduct the carbonylation process in the presence of hydrogen. Preferably at least part, most preferably all, of the said DME, emanates from the aforementioned etherification unit. However, according to an alternative embodiment of the present invention, at least a part of the dimethyl ether introduced into the carbonylation unit may also originate from a direct synthesis gas to dimethyl ether process. The reaction to produce dimethyl ether is exothermic and equilibrium controlled, as is the methanol synthesis reaction; however the reaction to produce dimethyl ether is more thermodynamically favourable than the methanol synthesis, under methanol synthesis reaction conditions. This has lead to close-coupling of these reactions in a single reactor as a means of increasing the potential yield per pass. This can be achieved by either adding acidity to the methanol catalyst, or by mixing an acidic catalyst with a methanol catalyst. Such processes are often referred to as direct synthesis e.g. Snamprogetti, SpA., DE 2 362 944, 1973 (G. Giorgio); DE 2 757 788, 1977 (G. Manara, B. Notari, V. Fattore); DE 3 220 547,1982 (G. Manara).

Suitable carbonylation catalyst(s) used in the present invention are acidic homogeneous or acidic heterogeneous catalysts. For example, the catalyst can be selected from homogeneous strong acid catalysts, e.g. phosphoric acid; and, heterogeneous analogues such as supported phosphoric acid and SAPO's catalysts. Other heterogeneous catalyst(s) include: zeolites; strong acid ionic resins catalysts; and mixtures thereof.

According to an embodiment of the present invention the carbonylation process for producing methyl ethanoate comprises reacting DME with carbon monoxide and preferably hydrogen under substantially anhydrous conditions, in the presence of a catalyst comprising a zeolite having at least one 8-member ring channel, said 8-member ring channel being interconnected with a channel defined by a ring with greater than or equal to 8 members, said 8-member ring having a window size of at least 2.5 Angstroms x at least 3.6 Angstroms and at least one Brønsted acid site and wherein the zeolite has a silica:X₂O₃ ratio of at least 5, wherein X is selected from aluminium, boron, iron, gallium and mixtures thereof, such as mordenite or ferrierite.

The feed to the carbonylation unit may comprise substantially pure carbon monoxide (CO), for example, carbon monoxide typically provided by suppliers of industrial gases, or it may contain impurities that do not interfere with the conversion of the DME to the target methyl ethanoate, such as nitrogen, helium, argon, methane and/or carbon dioxide. For example, the feed may comprise CO that is typically made commercially by removing hydrogen from synthesis gas via a cryogenic separation and/or use of a membrane.

In fact, according to a preferred embodiment of the present invention, the carbon monoxide feed contains substantial amounts of hydrogen. For example, the feed may be what is commonly known as synthesis gas, and is preferably derived from the above synthesis gas generation unit.

As indicated, the carbonylation process takes place in the presence of both carbon monoxide and hydrogen; preferably, the ratio of carbon monoxide: hydrogen is in the range 1:3 to 15:1 on a molar basis, such as 1:1 to 10:1.

Zeolites, both natural and synthetic are microporous crystalline aluminosilicate materials having a definite crystalline structure as determined by X-ray diffraction. The chemical composition of zeolites can vary widely but they typically consist of SiO₂ in which some of the Si atoms may be replaced by tetravalent atoms such as Ti or Ge, by trivalent atoms such as Al, B, Ga, Fe or by bivalent atoms such as Be, or by a combination thereof. A zeolite is comprised of a system of channels which may be interconnected with other channel systems or cavities such as side-pockets or cages. The channel systems are uniform in size within a specific zeolite and may be three-dimensional but are not necessarily so and may be two-dimensional or one-dimensional. The channel systems of a zeolite are typically accessed via 12-member rings, 10-member rings or 8 member rings. The specific zeolite mentioned hereinabove contains at least one channel which is defined by an 8-member ring. Preferred zeolites are those which do not have side-pockets or cages within the zeolite structure. The Atlas of Zeolite Framework Types (C. Baerlocher, W. M. Meier, D. H. Olson, 5th ed. Elsevier, Amsterdam, 2001) in conjunction with the web-based version (http://www.iza-structure.org/databases/) is a compendium of topological and structural details about zeolite frameworks, including the types of ring structures present in the zeolite and the dimensions of the channels defined by each ring type. For the purposes of the present invention, the term 'zeolite' also includes materials having a zeolite-type structure such as delaminated porous crystalline oxide materials such as ITQ-6 and pillared layered oxide materials such as ITQ-36.

The process of the present invention preferably employs a zeolite having at least one channel defined by an 8-member ring of tetrahedrally co-ordinated atoms (tetrahedra) with a window size having a minimum dimension of 2.5 Angstroms x 3.6 Angstroms. The 8-member ring channel is interconnected with at least one channel defined by a ring with equal to or greater than 8 members, such as 10 and/or 12 members. The interconnected 8-, 10, and 12- member ring channels provide access to Brønsted acid sites contained in the 8-member ring channels to enable the carbonylation of the DME to proceed at acceptable rates.

The zeolite for use in the present invention may consist of interconnected channels defined solely by 8-member rings, such as zeolites of framework type CHA, for example, chabazite and framework type ITE, for example ITQ-3. Preferably, however, the zeolite has at least one channel formed by an 8-member ring and at least one interconnecting channel defined by a ring with greater than 8 members, such as a 10, and/or 12 member ring. Non-limiting examples of zeolites having 8- member ring channels and interconnecting larger ring channel systems include zeolites of framework type MOR, for example, mordenite, FER, such as ferrierite and ITQ6, OFF, for example, offretite, GME, for example Gmelinite, MFS, such as ZSM-57, EON such as ECR-1 and ETR such as ECR-34. Preferably, the zeolites for use in the process of the present invention have at least one 8-member ring channel interconnected with at least one 12-member ring channel, such as those of framework type MOR, OFF and GME, for example, mordenite, offretite and gmelinite.

The aperture (pore width) of an 8-member ring channel of the zeolite has a minimum dimension of 2.5 x 3.6 Angstroms. Channel dimensions of zeolite framework types may be found, for example, in the *Atlas of Zeolite Framework Types.* In addition, M.D. Foster, I. Rivin, M.M.J. Treacy and O. Delgado Friedrichs in "A geometric solution to the largest-free-sphere problem in zeolite frameworks" Microporous and Mesoporous Materials 90 (2006) 32-38, have used Delaunay triangulation methods applied to known zeolite frameworks and have tabulated the largest free-sphere diameters for diffusion along the three principal crystallographic directions for the 165 zeolite frameworks that are currently listed in the *Atlas of Zeolite Framework Types.* Ring window sizes may be modified by suitable atomic substitutions that change bond lengths and bond angles of the tetrahedrally co-ordinated atoms and the bridging oxygens.

A partial listing of zeolite framework types having at least one interconnected 8 member ring channel of minimum dimension of 2.5 x 3.6 Angstroms taken from *The Atlas of Zeolite Framework Types* is given below:

| | | | | |
|---|---|---|---|---|
| MOR | Mordenite | 12 (6.5 x 7.0Å) | 8 (3.4 x 4.8Å) | 8 (2.6 x 5.7Å) |
| OFF | Offretite | 12 (6.7 x 6.8Å) | 8 (3.6 x 4.9Å) | |
| FER | Ferrierite | 10 (4.2 x 5.4Å) | 8 (3.5 x 4.8Å) | |
| CHA | Chabazite | 8 (3.8 x 3.8Å) | | |
| ITE | ITQ3 | 8 (3.8 x 4.3Å) | 8 (2.7 x 5.8Å) | |
| GME | Gmelinite | 12 (7.0 x 7.0Å) | 8 (3.6 x 3.9Å) | |
| ETR | ECR-34 | 18 (10.1Å) | 8 (2.5 x 6.0Å) | |
| MFS | ZSM-57 | 10 (5.1 x 5.4Å) | 8 (3.3 x 4.8Å) | |
| EON | ECR-1 | 12 (6.7 x 6.8Å) | 8 (3.4 x 4.9Å) | 8 (2.9 x 2.9Å) |

Zeolites are available from commercial sources. Alternatively they may be synthesized using known techniques. In general, synthetic zeolites are prepared from aqueous reaction mixtures comprising sources of appropriate oxides. Organic directing agents may also be included in the reaction mixture for the purpose of influencing the production of a zeolite having the desired structure. After the components of the reaction mixture are properly mixed with one another, the reaction mixture is subjected to appropriate crystallization conditions. After crystallization of the reaction mixture is complete, the crystalline product may be recovered from the remainder of the reaction mixture. Such recovery may involve filtering the crystals, washing with water followed by a calcination treatment at high temperature. The synthesis of zeolites is described in numerous references. For example, zeolite Y and its synthesis is described in US 3,130,007, zeolite ZSM-23 is described in US 4,076,842 and J.Phys. Chem. B, 109, 652-661 (2005), Zones, S.I. Darton, R.J., Morris, R and Hwany, S-J; ECR-18 is described in Microporous Mesoporous Mat., 28, 233-239 (1999), Vaughan D.E.W. & Strohmaier, K.G.; Theta-1 is described in Nature, 312, 533-534 (1984). Barri, S.A.I., Smith W.G., White, D and Young, D.; Mazzite is described in Microporous Mesoporous Mat., 63, 33-42 (2003), Martucci, A, Alberti, A, Guzmar-Castillo, M.D., Di Renzo, F and Fajula, F.; Zeolite L is described in Microporous Mesoporous Mat., 76, 81-99 (2004), Bhat, S.D., Niphadkair, P.S., Gaydharker, T.R., Awate, S.V., Belhekar, A.A. and Joshi, P.N and also in J. Ind. Eng. Chem. Vol. 10, No. 4 (2004), 636-644, Ko Y.S, Ahn W.S and offretite is described in Zeolites 255-264, Vol. 7, 1987 Howden M.G.

The zeolite catalyst for use in the process of the present invention is used in the acid form, generally referred to as the 'H' form of the zeolite, for example, H-mordenite, H-ferrierite. Other forms of the zeolite, such as the NH₄ form can be converted to the H-form, for example, by calcining the NH₄ form at elevated temperature. The acid form of a zeolite will possess Brønsted acid (H⁺) sites which are distributed among the various channel systems in the zeolite. For example, H-mordenite has H⁺ sites located in the 12 member ring channels and in the 8 member ring channels. The number or concentration of H⁺ species residing in any particular channel system can be determined by known techniques such as infra-red and NMR spectroscopic techniques. Quantification of Brønsted acidity by FTIR and NMR spectroscopy is described, for example, in Makarova, M.A., Wilson, A.E., van Liemt, B.J., Mesters, C. de Winter, A.W., Williams, C. Journal of Catalysis 1997, 172, (1), 170. The two types of channels in H-Mordenite (defined by 12 member rings and 8 member rings) give rise to at least two bands associated with the hydroxyl region of H-mordenite, one corresponding to vibration into the larger pores and the other, at a lower frequency, vibrating into the smaller pores. It has been shown that there is a correlation between the number of H⁺ sites located in an 8-member ring channel and the carbonylation rate whereas no such correlation has been observed for 12-member ring channels. It has been found that carbonylation rates increase in parallel with the number of H⁺ sites within 8 member ring channels. In contrast, no correlation is evident with the number of H⁺ sites within 12 member ring channels. The number of H⁺ sites within 8-member ring channels can be controlled by replacement of the H⁺ with metal cations such as Na⁺ or Co²⁺ using known ion-exchange techniques.

The chemical composition of a zeolite may be expressed as involving the molar relationship:

SiO₂ : X₂O₃

wherein X is a trivalent element, such as aluminium, boron, iron and/or gallium, preferably aluminium. The SiO₂ : X₂O₃ ratio of a given zeolite is often variable. For example, it is known that mordenite can be synthesized with SiO₂ : Al₂O₃ ratios of 6 to 90 or greater, zeolite Y, from about 1 to about 6, chabazite from about 2 to 2000 and gmelinite may be synthesised with SiO₂ : Al₂O₃ ratios of greater than 4. In general, the upper limit of the SiO₂ : X₂O₃ ratio is unbounded, for example, the zeolite ZSM-5. The specific zeolite mentioned hereinabove have a SiO₂ : X₂O₃ molar ratio of at least 5, preferably in the range 7 to 40, such as 10 to 30. Suitably, the SiO₂ : X₂O₃ molar ratio is less than or equal to 100. Particular SiO₂ : X₂O₃ ratios can be obtained for many zeolites by dealumination (where X is Al), by standard techniques using high temperature steam treatment or acid washing.

In one embodiment the carbonylation catalyst is composed of mordenite or ferrierite, or mixtures or combinations of the two, either per se (i.e., in the acid form, generally referred to as H-mordenite and H-ferrierite), or optionally ion-exchanged or loaded with one or more metals such as copper, nickel, iridium, rhodium, platinum, palladium, or cobalt. Mordenite catalysts may, in addition to silicon and aluminum atoms, contain further elements in the zeolite framework, particularly gallium and/or iron. Ferrierite catalysts may, in addition to silicon and aluminum atoms, contain further elements in the zeolite framework, particularly boron, gallium and/or iron. Framework modifier elements to both types of catalysts may be introduced to the framework by any conventional means. Where a framework modifier element is used in either a mordenite or ferrierite catalyst, the catalyst suitably has a ratio of silica to the oxide of the framework modifier element would be from about 10:1 1 to about 100:1. T-atom incorporation where T is B, Ga or Fe into zeolites of the ferrierite structure is disclosed in Melian-Cabrera et al., Catalysis Today 110 (2005) 255-263; Shawki et al., EP (Application) 234,766 (1987), Sulikowski et al., J. Chem. Soc., Chem. Comm., 1289 (1989); Borade et al., J. Chem. Soc., Chem.Comm., 2267 (1996); Jacob et al., Zeolites 430 (1993) Vol. 13. T-atom incorporation into zeolites of the mordenite structure where the T-atom is Ga or Fe is disclosed in Smith, WO 05/085162.

Mordenite (commonly available as Na-mordenite, NH₄-mordenite or H-mordenite) is a member of the aluminosilicate zeolite class of minerals. The formula of mordenite in its Na-form is usually given as Na(AlSi₅O₁₂).3H₂O or (Na₂,Ca,K₂)Al₂Si₁₀O₂₄.7H₂O. It is available from a number of commercial sources of such materials. H-Mordenite has elliptical 6.5 x 7.0 Å channels (12 member rings with window openings running in the crystallographical c-direction). It also has a system of smaller channels running perpendicular to the 12 member ring channels (and running in the *b*-direction). These small channels consist of 3.4 x 4.8 Å channels having 8 member ring windows of these dimensions. The mordenite structure also possesses a zig-zag Y-branching of the pore structure due to twisted 8 member rings (in the crystallographic c-direction). This results in a distorted window to each side of the Y-branching of 2.6 x 5.7 Å. Ferrierite is another member of the aluminosilicate zeolite class of minerals, also available in the Na-, NH₄- and H- forms. In the Na-form its formula generally is given as Na_{0.8}K_{0.2}MgSi₁₅Al₃O₃₆.9H₂O or (Mg,Na₂,K₂,Ca)₃₋₅Mg[Al₅₋₇Si_{27.5-31}O₇₂].18H₂O. It, too, is available from various commercial sources. Additional information on these and other materials can be found on the website of the International Zeolite Association, www.iza-online.org. H-Ferrierite has 4.2 x 5.4 10-member ring channels running in the crystallographic *c*-direction and 3.5 x 4.8 8-member ring channels running in the *b*-direction.

The reaction is to be conducted substantially in the absence of water when using the specific zeolite mentioned hereinabove, for example by drying the zeolite catalyst before beginning the operation, for example, by preheating to 400- 500°C.

According to an embodiment of the present invention, the carbonylation process is run at temperatures at or below about 600°C, that is, at temperatures of from about 200 to about 600 °C, preferably from about 250 to about 400 °C. One feature of the process is that, surprisingly, the carbonylation of dimethyl ether (DME) to methyl ethanoate using mordenite zeolite based catalysts and in the substantial absence of water can be performed with very high selectivities.

According to an embodiment of the present invention total operating pressures in the carbonylation process are from about 1 MPa to about 20 MPa, preferably from about 1 MPa to about 15 MPa and most preferably from about 2.5 MPa to about 10 MPa; preferably with carbon monoxide pressures greater than 1 MPa and dimethyl ether pressures below 0.5 MPa

The zeolite carbonylation process is carried out under substantially anhydrous conditions, i.e. in the substantial absence of water. The carbonylation of dimethyl ether to methyl ethanoate does not generate water in-situ. Water has been found to inhibit the carbonylation of dimethyl ether to form methyl ethanoate. This is in comparison to prior art processes, in which dimethyl ether was a co-feed, and in which water was also fed to the reaction. Water is thus kept as low as feasible, in order to allow the desired reaction to proceed best. To accomplish this, the ether and carbon monoxide reactants and the catalyst are preferably dried prior to introduction into the process.

The carbonylation process may be run as either a continuous or a batch process, with continuous processes typically preferred. Essentially, the heterogeneous process is a gas-phase operation, with reactants being introduced in either liquid or gaseous phase, and products withdrawn as gases. As desired, the reaction products may subsequently be cooled and condensed. The heterogeneous catalyst may be used as convenient, in either a fixed bed or a fluidized bed. In operating the process, unreacted starting materials may be recovered and recycled to the carbonylation unit. The product methyl ethanoate is then fed to the hydrogenation unit; however, some methyl ethanoate may also be recovered and sold as such, or may be forwarded to other chemical process units as desired. Preferably, the entire reaction product may be sent to the hydrogenation unit for conversion of the methyl ethanoate to methanol and ethanol.

According to a preferred embodiment of the present invention, the methyl ethanoate stream exiting the carbonylation unit is then purified to remove ethanoic acid and water, before being introduced into the hydrogenation unit. After purification and before introduction into the hydrogenation unit, the alkyl ester stream contains preferably less than 5 wt% of ethanoic acid, more preferably less than 1 wt%, even more preferably less than 0.1wt% and most preferably less than 100ppm by weight. The alkyl ester feed is also preferably free from residual carboxylic acid and has a total acid number (TAN) of less than 100, more preferably less than 50 and most preferably less than 5 (where, according to the present invention, TAN is the amount (mg) of Potassium Hydroxide needed to fully neutralize 1g of the sample material).

After purification and before introduction into the hydrogenation unit, the alkyl ester stream contains preferably less than 20 wt% of water, more preferably less than 2 wt%, most preferably less than 0.2 wt%.

These said purification treatments are especially advantageous when a copper-based catalyst is employed in the hydrogenation unit.

Furthermore, the alkyl ester feed is preferably vapourised prior to contacting the hydrogenation catalyst, by either heating the alkyl ester in a separate vapouriser prior to having contact with the hydrogen or, by heating the alkyl ester together with the hydrogen (e.g. either in a separate vessel or on a pre-bed to the hydrogenation unit). The feed mixture including recycles entering the hydrogenation unit (e.g. the hydrogen together with the alkyl esters), is preferably introduced at greater than 10°C above its dew point temperature, and more preferably at greater than 20°C above its dew point temperature. For the purposes of the present invention and appending claims, the 'dew point temperature' is defined as being a threshold temperature. For example, for a given mixture, at a given pressure, if the system temperature is raised to above the dew point temperature, the mixture will exist as a dry gas.

According to the present invention, methyl ethanoate is introduced into a hydrogenation unit together with hydrogen to produce a stream comprising ethanol and methanol. In addition to the production of ethanol, the hydrogenation process also produces other reaction products such as ethyl ethanoate (e.g. trace amounts of methane, ethane, diethyl ether, water and ethanal) and unreacted starting materials e.g. unreacted methyl and/or ethyl ethanoate (from recycle) and unreacted hydrogen.

When methyl ethanoate is used as a feed for the hydrogenation process, it also produces ethyl ethanoate by trans-esterification. The proportion of ethyl ethanoate present in the exit stream will be determined by the nature of the catalyst and the degree of conversion. The proportion of ethyl ethanoate may be further increased, if desired, by introducing an acidic function into the catalyst bed to promote 'in situ' trans-esterification. This transesterification is advantageous, as it reduces the amount of unreacted methyl ethanoate present in the reactor effluent- since the methyl ethanoate forms a close boiling point mixture with the methanol, and is thus carried with the methanol recycles to other process stages such as the etherification unit and/or as a recycle to the hydrogenation unit. More preferably the quantities of these fluxes are minimised.

Preferably the hydrogenation unit is operated at high conversion of methyl (and recycled ethyl ethanoate) to ethanol, such as greater than 50%, more preferably greater than 75%, more preferably still greater than 90% and most preferably greater than 95%.

It is has also been found to be an advantage of this process, that the selectivity of the methyl and/or ethyl ethanoate hydrogenation to ethanol process can be further increased, at the expense of undesirable by-products, such as the aforementioned alkanes (e.g. ethane and methane).

Preferably, at least a part, more preferably all, of the hydrogen fed into the hydrogenation unit emanates from the synthesis gas generation procedure (i.e. it is obtained during the aforementioned carbon monoxide/hydrogen separation), where, if need be, the hydrogen content can be further increased by subjecting the said synthesis gas to a water gas shift reaction and a subsequent hydrogen separation.

Alternatively the hydrogen stream may originate from a variety of other chemical processes, including ethene crackers, styrene manufacture and catalytic reforming. However, it is known that the main commercial processes for purposeful generation of hydrogen are autothermal reforming, steam reforming and partial oxidation of hydrocarbon feedstocks such as natural gas, coal, coke, deasphalter bottoms, refinery residues and biomass. Hydrogen may also be produced by electrolysis of water.

The overall choice of technology for producing hydrogen is generally determined by the following economic considerations and factors:
i. feedstock cost
ii. feedstock availability
iii. capital cost
iv. local energy and operating costs; and
v. environmental considerations

The catalyst(s) employed in the hydrogenation unit is selected from any of the following:
(i) a precious metal based catalyst, comprising of at least one noble metal from Group VIII of the periodic table (CAS version, for example iron, ruthenium, osmium, cobalt, rhodium, iridium, nickel, palladium, platinum) and at least one of the metals chosen from rhenium, tungsten and/or molybdenum; and optionally an additional metal, that is capable of alloying with said Group VIII noble metal; or
(ii) a copper-based catalyst (for example a copper chromite or a mixed copper metal oxide based catalyst wherein the second metal can be Cu, Zn, Zr or Mn).

According to a preferred embodiment of the present invention, the catalyst(s) employed in the hydrogenation unit is a copper based catalyst; whereby the preferred copper based catalyst is a supported catalyst which comprises copper, and preferably promoters, such as cobalt and/or manganese and/or chromium.

All of the aforementioned catalysts may advantageously be supported on any suitable support known to those skilled in the art; non-limiting examples of such supports include: carbon; silica; titania; clays; aluminas; zinc oxide; zirconia; and mixed oxides. Preferably, the palladium-based catalyst is supported on carbon. Preferably, the copper-based catalyst is supported on zinc oxide.

According to a preferred embodiment of the present invention, the catalyst(s) employed is heterogeneous.

The hydrogenation process may be operated in a gas phase, or a mixed gas/liquid phase regime. The mixed gas/liquid phase is where a proportion of the substrate (consisting of methyl and/or ethyl ethanoate and ethyl ethanoate) to be hydrogenated is in the liquid phase. The hydrogenation can be conducted in batch or semi continuous or continuous mode. Continuous mode of operation is the most preferred.

The hydrogenation reaction can be conducted in adiabatic or isothermal mode; where adiabatic mode of operation is preferred. Suitable units include single, or a plurality, of adiabatic bed reactors which can be used in series or in parallel. For reactors utilised in series, heat exchangers and/or intercoolers and/or additional reactant and/or recycle of intermediates can be employed in between successive reactors to control the reaction temperature. The preferred adiabatic temperature rise is less than 50°C, preferably less than 25°C and most preferably less than 10°C. The preferred use of adiabatic reactors is in series. The adiabatic reactors may be operated at different temperatures depending on composition of the individual reactor feeds.

The hydrogenation can also be conducted in multi-tubular reactors in which case a cooling/heating medium is circulated around the tubes to control the temperature. For exothermic reactions, as such, there will be a radial temperature gradient in the reactor, the preferred gradient is less than 50°C, more preferably less than 25°C and most preferably less than 10°C. The preferred flow regime in this type of reactor is turbulent rather than laminar; this corresponds to a Reynolds number greater than 2100 (where the velocity is approximated by velocity in an unpacked tube).

The hydrogenation reaction can also be conducted in other unit types such as fluidised bed, spinning basket and buss loop or heat exchanger reactors. A mixed liquid/gas phase hydrogenation reaction can be conducted with co-flow or counterflow of the hydrogen and vapour to the liquid (e.g. a bubble reactor). The preferred mode of operation of gas/liquid reactors is co-flow, also known as trickle bed operation; this can be conducted in at least one tubular and/or multi-tubular reactor in series. The hydrogenation reaction may change from a mixed gas/liquid phase to a fully vapour phase reaction, as the reaction proceeds down the reactor. The mixed phase hydrogenation can also be conducted in other types of reactors, or within a combination of different reactors, for example in a slurry or stirred tank reactor with, or without, external circulation and optionally operated as a cascade or stirred tanks, a loop reactor or a Sulzer mixer-reactor.

The hydrogenation unit preferably operates at a temperature of greater than 150 °C, but less than 290 °C.

According to a preferred embodiment of the present invention the reaction temperature is greater than 150°C, preferably greater than 170°C and most preferably greater than 190°C; and less than 250°C, preferably less than 230°C and most preferably less than 220°C.

The hydrogenation reaction may be operated at a pressure of greater than 1 MPa, more preferably at a pressure of greater than 5 MPa and most preferably at a pressure of greater than 7 MPa; and at a pressure of less than 15 MPa, more preferably at a pressure less than 13 MPa and most preferably at a pressure less than 9 MPa.

According to an embodiment of the present invention, when the hydrogenation unit(s) is operated with a copper-based catalyst, the feed mixture introduced into the unit is always above its dew point temperature.

For the purposes of the present invention and appending claims, the 'dew point temperature' is defined as being a threshold temperature. For example, for a given mixture, at a given pressure, if the system temperature is raised to above the dew point temperature, the mixture will exist as a dry gas. Likewise below the dew point temperature, the mixture will exist as a vapour containing some liquid.

The Gas Hourly Space Velocity (GHSV- which is defined as the rate of gas volume, per catalyst volume per hour, at standard temperature and pressure), for continuous operation may be in the range 50 to 50,000 h⁻¹, preferably from 1,000 to 30,000 h⁻¹ and most preferably from 2,000 to 9,000 h⁻¹.

The ester liquid substrate introduced into the hydrogenation unit preferably has a Liquid Hourly Space Velocity (LHSV- which is defined as the rate of liquid volume, per catalyst volume, per hour, at standard temperature and pressure), which may be less than 10hr⁻¹, more preferably less than 5hr⁻¹ and most preferably less than 3hr⁻¹; for example, a typical LHSV for normal operation is approximately 1hr⁻¹.

For the purposes of calculating the GHSV and/or the LHSV of the present invention, when the reactors are arranged in series, said "catalyst volume" refers to the combined volume of the catalyst in all of the reactors in series.

According to a preferred embodiment of the present invention, the molar ratio of hydrogen to [methyl ethanoate and ethyl ethanoate] that is introduced into the hydrogenation unit is greater than 2:1, preferably the molar ratio is greater than 4:1 and most preferably the molar ratio is greater than 5:1; and is less than 100:1, preferably less than 50:1 and most preferably less than 15:1.

According to a preferred embodiment of the present invention, the effluent exiting the hydrogenation reactor, (or the last in a train of reactors), is subjected to a separation stage, e.g. cooling the effluent and then feeding the cooled effluent to a flash vessel (wherein the said effluent can be both cooled and/or flashed several times). After this treatment, a vapour stream containing the majority of the hydrogen and inert gases (including light alkanes; nitrogen; CO; and CO2) in the effluent is recovered for recycle to the hydrogenation unit(s). The concentration of said inert gases can be controlled by diverting a proportion of the gas recycle stream to purge.

According to the present invention, the liquid stream exiting the hydrogenation unit is then subjected to a separation stage (e.g. distillation), whereby a fraction comprising alcohol(s) (i.e. methanol and ethanol) is separated and recovered and at least a part, preferably all, of the recovered methanol is used as the feed for the etherification unit. The ethanol is recovered as the desired product.

Additionally, another separation embodiment at the liquid exit of the hydrogenation unit consists of isolating methyl ethanoate/methanol close boiling point mixture and/or the ethyl ethanoate/ethanol azeotropes and recycling said isolated compounds to the hydrogenation unit. Alternatively, the methyl ethanoate/methanol close boiling point mixture can be passed to the etherification unit. As a further embodiment to the present invention, the methyl ethanoate can be hydrogenated by co feeding with stoichiometric amount of hydrogen to the methanol unit.

It should be noted that whilst all of the aforementioned temperature and pressure operating conditions form preferred embodiments of the present invention, they are not, by any means, intended to be limiting, and the present invention hereby includes any other pressure and temperature operating conditions that achieve the same effect.

## Claims

1. A process for the conversion of methanol to ethanol, **characterised by** the following steps:
1. introducing methanol into an etherification unit to produce dimethyl ether,
2. introducing at least a part of the dimethyl ether from step 1, together with carbon monoxide and optionally hydrogen, into a carbonylation unit, in the presence of an acidic homogeneous or heterogeneous carbonylation catalyst, to produce methyl ethanoate,
3. introducing at least a part of the methyl ethanoate from step 2, together with hydrogen, into a hydrogenation unit, to produce methanol and ethanol,
4. optionally recycling at least part of the methanol from step 3, into the etherification unit of step 1, and
5. recovering ethanol from step 3.

2. A process for the conversion of a hydrocarbon feedstock into ethanol, wherein the hydrocarbons are first converted into synthesis gas, which is subsequently converted into ethanol, **characterised by** the following steps:
1. introducing a hydrocarbon feedstock, into a synthesis gas generation unit, in order to produce a mixture of carbon oxide(s) and hydrogen (synthesis gas),
2. introducing at least a part of the carbon oxide(s) and hydrogen, from step 1, into a methanol synthesis unit, to produce methanol,
3. introducing at least a part of the methanol from step 2, into an etherification unit to produce dimethyl ether,
4. introducing at least a part of the dimethyl ether from step 3, together with carbon monoxide and optionally hydrogen, into a carbonylation unit, in the presence of a heterogeneous carbonylation catalyst, to produce methyl ethanoate,
5. introducing at least a part of the methyl ethanoate from step 4, together with hydrogen, into a hydrogenation unit, to produce methanol and ethanol,
6. optionally recycling at least part of the methanol, from step 5, into the etherification unit of step 3, and
7. recovering the ethanol from step 5.

3. A process according to any of the preceding claims, wherein the catalyst(s) in the methanol synthesis unit is a low pressure copper catalyst(s), composed of zinc oxide, copper oxide and a promoter.

4. A process according to any of the preceding claims, wherein the catalyst(s) employed in the etherification unit is selected from the following: zeolites; strong acid ion exchange resins; supported heteropolyacids; or mixtures thereof.

5. A process according to any of the preceding claims, wherein during the methanol etherification process water is also produced; this said water is selectively removed prior to the introduction of the dimethyl ether into the carbonylation unit.

6. A process according to any of the preceding claims, wherein the carbonylation process is conducted in the presence of hydrogen.

7. A process according to any of the preceding claims, wherein the carbonylation process is performed in the presence of an acidic homogeneous or acidic heterogeneous catalyst, and is selected from homogeneous strong acid catalyst(s), e.g. phosphoric acid; heterogeneous analogues - such as supported phosphoric acid and SAPO's catalysts; and, heterogeneous catalysts including zeolites, strong acid ionic resins catalysts; and/or mixtures thereof.

8. A process according to any of the preceding claims, wherein the carbonylation process is performed in the presence of a catalyst comprising a zeolite having at least one 8-member ring channel.

9. A process according to Claim 8, wherein the zeolite catalyst for use in the carbonylation process is used in the acidic form.

10. A process according to either Claim 8 or 9, wherein the carbonylation catalyst is composed of mordenite or ferrierite, or mixtures or combinations of the two.

11. A process according to any of the preceding claims, wherein the carbonylation process is performed under substantially anhydrous conditions.

12. A process according to any of the preceding claims, wherein the carbonylation process is run at temperatures of from 200 to 600 °C, preferably from 250 to 400 °C.

13. A process according to any of the preceding claims, wherein the hydrogenation unit is operated at greater than 50%, more preferably greater than 75%, more preferably still greater than 90% and most preferably greater than 95% conversion of alkyl esters to ethanol.

14. A process according to any of the preceding claims, wherein the catalyst(s) employed in the hydrogenation unit is a copper-based catalyst, such as copper chromite or a mixed copper metal oxide based catalyst wherein the second metal can be Cu, Zn, Zr or Mn.

15. A process according to any of the preceding claims, wherein the alkyl ester stream entering into the hydrogenation reactor contains less than 5 wt%, preferably less than 1 wt%, more preferably less than 0.1wt% and most preferably less than 100ppm by weight of ethanoic acid.

16. A process according to any of the preceding claims, wherein the alkyl ester stream entering into the hydrogenation unit contains less than 20 wt%, more preferably less than 2 wt%, most preferably less than 0.2 wt% of water.

17. A process according to any of the preceding claims, wherein the hydrogenation unit is operated at a temperature that is greater than 150°C., preferably greater than 170°C and most preferably greater than 190°C; and less than 250°C, preferably less than 230°C and most preferably less than 220°C.

18. A process according to any of the preceding claims, wherein the hydrogenation unit is operated at a pressure of greater than 1 MPa, more preferably at a pressure of greater than 5 MPa and most preferably at a pressure of greater than 7 MPa; and at a pressure of less than 15 MPa, more preferably at a pressure less than 13 MPa and most preferably at a pressure less than 9 MPa.

19. A process according to any of the preceding claims, wherein the methanol exiting the hydrogenation unit is isolated and then introduced into the etherification unit.

20. A process according to any of the preceding claims, whereby a methyl ethanoate/methanol close boiling point mixture is isolated from the stream exiting the hydrogenation unit and is then introduced into the etherification unit.

21. A process according to any of the preceding claims, whereby ethyl ethanoate/ethanol azeotropes are isolated from the stream exiting the hydrogenation unit and are then introduced into the hydrogenation unit.
